# EUROPEAN PATENT APPLICATION

(11) **EP 2 256 704 A1**
(43) Date of publication of application: **01.12.2010**
(21) Application number: 09725573.1
(22) Date of filing: 13.03.2009
(51) Int. Cl.: G07F 17/04, A61B 5/02

(54) **MULTIFUNCTIONAL EQUIPMENT FOR MEASURING VITAL DATA, PROVIDED WITH A CENTRALISED DATA PROCESSING SYSTEM**

(30) Priority: 26.03.2008 ES 200800847
(71) Applicant: Tuylek, S.L., 08349 Cabrera de Mar, Barcelona (ES)
(72) Inventor: PONS FERRÉ, Angel, E-08340 Vilassar de Mar Barcelona (ES)
(74) Representative: Urteaga Simarro, José Antonio
(86) International application number: PCT/ES2009/000144
(87) International publication number: WO 2009/118434

(57) **Abstract**

The invention relates to multifunctional equipment intended for measuring the vital data of a user, such as weight, height, body fat index and blood pressure. For this purpose, the equipment includes a platform for supporting the user, a device for emitting an ultrasonic beam and a receiver for receiving the reflected beam, a device for measuring blood pressure, a keyboard and screens for providing help and displaying the results, as well as payment means. The equipment also includes a digital fingerprint reader intended for the unequivocal identification of a user, together with an internal data processing device provided with inlet/outlet ports for connecting same to a computer associated with the equipment or to a remote computer forming a central database which can be accessed both from the first computer associated with the equipment and from other external computers over the Internet.

## Description

### Object of the Invention

The object of the invention refers to a multifunction device for measuring vital signs equipped with a centralised data processing system that offers essential innovative features and notable advantages compared to those which are known and used for the same purpose according to the current state of the technology.

In particular, the invention proposes the development of a piece of equipment with the ability to take a variety of conventional vital sign measurements for a user but also includes a device that reads and recognises fingerprints for the automatic and immediate identification of the user as well as an output for sending the data to a centralised database, such as a a computer, by means of an internal or external network, so that each user's history is more easily accessible to the user himself and to the physician or other health care professional responsible for his care.

The field of application of the invention includes the industrial sector dedicated to the production and installation of multifunction vital sign measurement devices equipped with functions to oversee and maintain the data generated.

### Background and summary of the invention

Experts in the field are aware of the existence in the market of machines capable of taking a user's vital signs. For example, the multifunction KEITO® devices owned by the applicant of this patent are machines for use by the general public that may be installed in places such as pharmacies and medical offices which have been designed to take a user's measurements such as height, weight, blood pressure, pulse, or body fat index. All of these machines are equipped with displays and printers for printing out the results of the measurements voice systems for guiding the user during the measurement process, keyboards for entering the data (if necessary), coin slots and card chip readers.

The user can activate the measurement system in one of three ways:
- By paying for the service in advance using the coin slot installed in the machine.
- By identifying himself using a card with a chip installed in it containing the user's available credit and a brief history of the user's measurements or
- By clicking on one of the keys on the machine specifically programmed for free operation of the machine.

However, for some years now the experts in the field have been seeking a system to universalise and extend the functionalities of these devices. To that end, all of the devices are equipped with card chip readers which make it possible to:
- Use the card as a means of identification;
- Forego the use of coins since the credit available to the user is stored in the card's memory.
   Depending on the type of card, it may offer unlimited use (e.g., clients of insurance companies and associations). The cards can also be used as a means of building customer loyalty (e.g., in pharmacies), giving the user of the card credit based on the purchases made at a particular establishment;

- Store the measurements in the card;
- When a user uses the machine, it prints a receipt using the equipment's built-in printer containing the history of the latest measurements taken along with the results of the most recent measurements which allows the user to control his weight and body fat index (e.g., people on a diet), height (growing children), or blood pressure (people with blood pressure problems);
- Finally, the use of a reader connected to a computer allows the physician to see the patient's historical measurements in order to monitor the evolution of the parameters.

However, despite the undeniable advantages of using cards with chips installed for the specified purpose, there are certain disadvantages, some of the most significant of which are summarised below:
- Price: while the price of an individual card may be low, the use of the system by thousands or millions of users involves a significant investment which is difficult to make in a non-standardised system;
- Usurpation: although the cards are intended to be personal and non-transferable, there is no guarantee that the card will always be used by its intended owner;
- Lost cards: when a card is lost, all of the historical measurement data is lost along with it.

However, the appearance of new technologies in the fields of user recognition and transmission of measurement data have made it possible to develop this invention with the clear purpose of providing effective solutions to the problems that currently exist in relation to this type of equipment. The proposed objective has been fully achieved by the multifunction measuring device that will be described below, the principal characteristics of which are contained in the characterisation portion of claim 1, enclosed herewith.

In essence, the invention makes it possible for a KEITO® type device to be used properly and with the complete assurance that it is being used by the right person. To this end, the equipment has been improved by replacing the card reader with a device for identifying the user consisting of a fingerprint-identifying device. The data generated on the user's fingerprints are sent to a central computer where they are compared with the information stored in the database, which then authorises the use of the equipment based on the results of the comparison; if affirmative, when the user is finished using the equipment the measurement data are sent to the central computer where they are stored along with a pre-determined number of prior measurements.

To do so, the measurement equipment includes a means for connecting the device to a storage and registration device such as a a computer at the installation site and other means for connecting to the Internet via a central computer in a remote location. Thanks to this storage capability, the data are accessible to the user and to medical professionals and others in charge of overseeing the patient's health.

### Brief description of the drawings

These and other characteristics and advantages of the invention will be made clearer from the following detailed description of an example of an embodiment of the invention which is provided merely for illustrative and non-limitative purposes, along with the enclosed drawings, where:

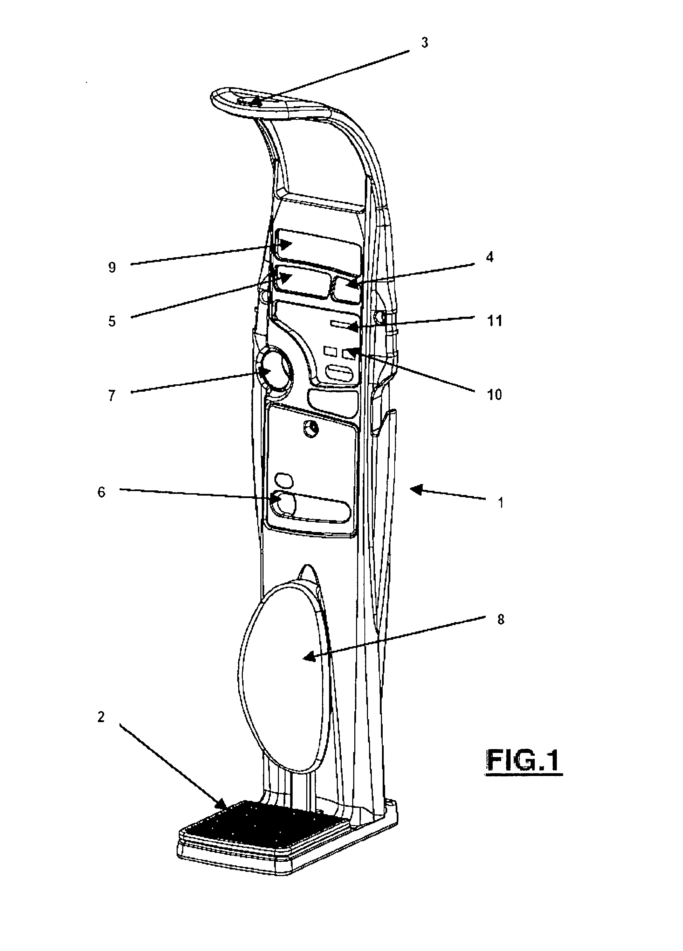

Figure 1 is an illustration of the multifunction device for measuring vital signs built according to the teachings of the present invention and Figure 1 is a schematic illustration of its operation.

### Description of a preferred form of embodiment

As mentioned above, a detailed description of the preferred form of embodiment of the invention will be given below with the help of the enclosed drawings, where numerical references are used to designate the parts or elements of the invention. Thus, according to the representation in Figure 1, which illustrates a general device designated with the numerical reference of 1, which shows a KEITO® type machine that takes a number of measurements such as the user's weight, height, blood pressure, etc. To this end, the conventional embodiment of the device includes a platform 2 comprising a scale, a system for sending 3 and receiving an ultrasonic beam to calculate height, a keyboard 4 for entering data as needed, a screen 5 to display the results, a printer 6, a device 7 associated with an opening where the user's wrist is introduced for the purpose of taking the user's blood pressure, an illustrated, back-lit help screen 9 and a slot 10 for inserting coins. In addition, there is a support 8 that can be used for taking a baby's measurements.

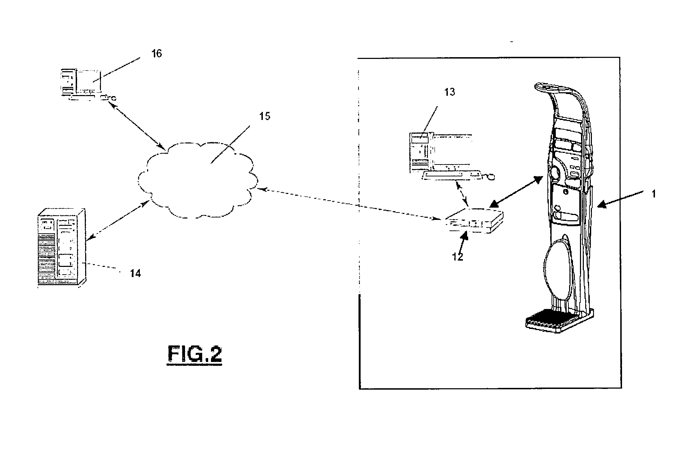

In addition to all of the functionalities mentioned above, the invention improves the equipment by including a fingerprint recognition device that guarantees the user's unequivocal identification. This device, which in the case of the embodiment shown in Figure 1 of the drawings has been rendered in the position shown with reference 11, may be positioned in any other way which guarantees its convenient operation by the person using the equipment. Moreover, as can be seen in Figure 2, the device also includes 12 internal media for processing the user's data equipped with input/output ports for connecting the equipment 1 to the database devices used to store the measurement data generated by the equipment and supply the data back to the equipment as necessary. These external devices may consist of a computer 13 installed in the place where the device is located with the use of an internal network or a computer 14 that serves as a central database which is accessed through an external network 15 such as the Internet, in a remote location, and which can also be accessed from the computer 13 located where the equipment 1 is installed or from other computers 16 via the network itself, managed by the user or by someone else in charge of caring for the user.

With a device such as the one described above, the use of the entire assembly is extremely simple and convenient because of the following functional characteristics. With the user standing on the platform 2 of the equipment 1, the fingerprint reader 11 is automatically activated, which will normally be signalled by means of a small light coming on (e.g., a LED diode, not shown) and the user will be advised to place his finger on the fingerprint reader 11. Once the fingerprint has been read, the information generated by the reader is sent via the network 15 to a computer 14 housing the central database, where the data received are compared with the data stored in the database. If the user is positively identified, a data package is sent back to the equipment 1 from the computer 14 containing the identification number, sex and age of the user, a list with some prior measurements (e.g., the last 20 measurements) and the order to take new measurements.

There are two different ways of activating the system. In the first form of activation from the equipment itself, once the fingerprint has been read and recognised, the the sequence mentioned above is carried out. If, however, the user's fingerprint is not recognised, the computer creates a temporary identification code linked to the fingerprint and sends an order to initiate the measurement cycle along with the temporary identification code. The equipment 1 initiates the measurement process and when it is over asks the user to enter certain information such as his age and sex. A receipt is then printed with the results of the measurements and the user is informed of the assigned code. The equipment 1 then contacts the central computer 14 and forwards the age, sex and temporary code of the user and the measurement results to be permanently stored in the database. The next time the same user's measurements are taken, the central computer 14 will provide the information to the equipment 1.

In the second form of activation, the cycle is initiated by the computer 14 which sends an order to the equipment 1 to read the user's fingerprint when the user's presence is detected. When the user places his finger in the reading device 11, the results of the reading are sent to the computer 13. When the information required for the system to function is entered (age and sex), this information along with the fingerprint reading are sent to the central computer 14 for storage by creating a record for that user and the corresponding identification code.

Obviously, a multifunction device for taking vital measurements of the kind proposed by this invention offers multiple advantages over other devices currently existing in the market, such as:
- Economy: the equipment referred to in the invention is much more economical due to the fact that it is much less expensive to include a fingerprint reader with each unit 1 than to issue chip-containing cards for each user;
- Agility: the data are accessible on line to health care professionals who need to monitor their patients;
- Convenience: users can use any piece of equipment located anywhere in the world and have easy access to their historical records;
- Reliability: the use of equipment 1 which includes fingerprint verification makes usurpation by other users very difficult;
- Safety and anonymity: the user must only enter his age, sex and fingerprint in order to use the equipment 1; no other information that could enable third parties to identify the user is required. In addition, in order to consult the data the user identification code must be entered along with prior measurements. Even if the receipt with results of the latest measurements is lost, a third party could access the historical measurements but could not identify the person

to whom they belong; once a new set of measurements is subsequently taken, the data are protected once again.

It is not considered necessary to expand any further upon this description in order for an expert in the field to understand the scope of the invention and the advantages derived therefrom or to carry out a practical embodiment of the object.

The above notwithstanding, given that the description refers only to an example of a preferred embodiment, it should be understood that multiple variations could be introduced that could affect the characteristics of the measurement equipment, the type of fingerprint identification device built into the equipment, the way in which the equipment is connected to the external databases or any other detail which does not alter the scope of the invention.

## Claims

1. A multifunction device for measuring vital signs equipped with a centralised data processing system, in particular a device (1) of the kind that is designed and built for installation in public establishments and other places frequently accessed for taking a user's measurements such as weight, height, body fat index or blood pressures, which device (1) includes a platform (2) for determining the user's weight, a device (3) for sending and receiving an ultrasonic beam used to measure the user's height, a device for taking blood pressure (7), a keyboard (4) for data entry, an opening (10) for inserting coins, a screen (5) for displaying results, a device (6) for printing receipts with the measurement results and the historical results based on a pre-determined number of prior measurements for the same user (8), a baby scales and a help screen (9) **characterised by** the inclusion of a fingerprint reading device (11) for scanning the user's fingerprints and by the inclusion of a data processing device (12) equipped with input/output ports for connection to a computer (13) associated with the place where the equipment is installed through a local network or with a computer (14) connected to the Internet (15) located in a remote position and usable as a central database for storing and recovering the results of the measurements taken by the device.

2. A device according to claim 1 **characterised by** the fact that it can be activated by the user once positioned on the platform (2) by scanning his fingerprint using the fingerprint recognition device (11) or which can be activated by a computer (13) associated with the equipment (1).
